# EUROPEAN PATENT SPECIFICATION

(11) **EP 4 569 102 B1**
(45) Date of publication and mention of the grant of the patent: **02.12.2026**
(21) Application number: 23757835.6
(22) Date of filing: 02.08.2023
(51) Int. Cl.: C12N 15/10, C12Q 1/6806, C12Q 1/6855

(54) **BARCODING OF NUCLEIC ACIDS FROM SINGLE CELLS**
BARCODIERUNG VON NUKLEINSÄUREN AUS EINZELNEN ZELLEN
CODAGE À BARRES DES ACIDES NUCLÉIQUES À PARTIR DE CELLULES INDIVIDUELLES

(30) Priority: 12.08.2022 EP 22190128
(43) Date of publication of application: 18.06.2025
(73) Proprietor: Miltenyi Biotec B.V. & Co. KG, 51429 Bergisch Gladbach (DE)
(72) Inventor: WAHL, Matthias Bernhard, 51429 Bergisch Gladbach (DE)
(74) Representative: Becker, Kristin
(86) International application number: PCT/EP2023/071374
(87) International publication number: WO 2024/033174

(56) References cited:
- WO-A1-2019/157529
- WO-A1-2021/247593
- WO-A2-2020/112604

## Description

### Field of the invention

The current invention relates to the field of single cell analysis and nucleic acid barcoding.

### Background of the invention

Methods for analyzing nucleic acids on single cell level are increasingly exploited in biological and biomedical research. Such methods allow understanding the heterogeneity of tissues or cell populations, and can be utilized to identify sub-populations of cells implicated in disease.

To facilitate single cell analysis and enable multiplexing current state in the art workflows include two essential features: partitioning of the each single cell and barcoding of cell specific target nucleic acids.

Current methods for analyzing single biological particles, including single cells, use either droplets (water-in-oil emulsions) or small vessels for partitioning these particles and subsequently incorporating a specific barcode into the target nucleic acid molecules within these partitions. Based on that, barcoded target nucleic acid molecules from one cell can be distinguished from target nucleic acid molecules from another cell during a cell analysis step, such as next generation sequencing. One example is the analysis of different mRNA transcripts expressed in individual cells in a highly parallel fashion.

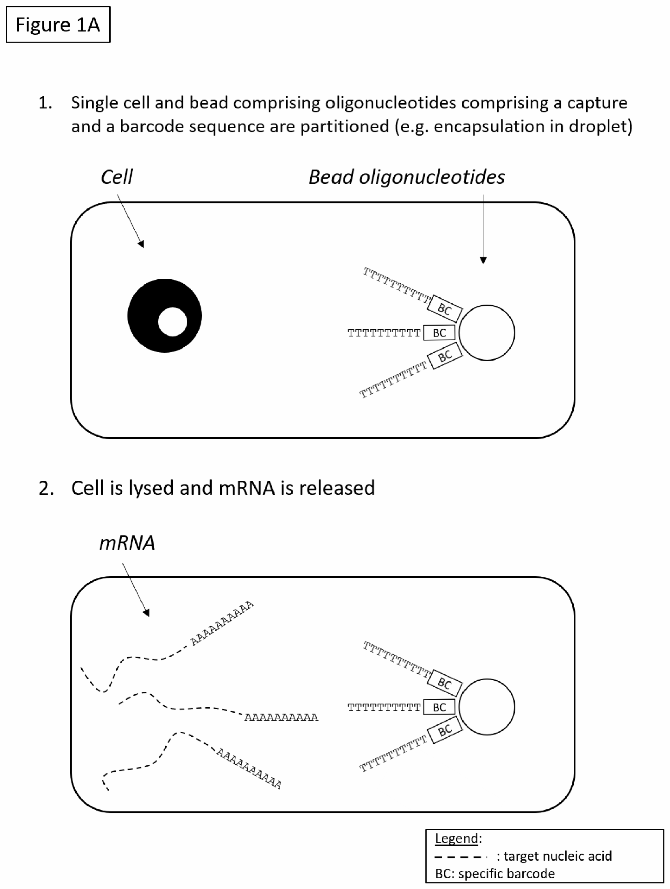

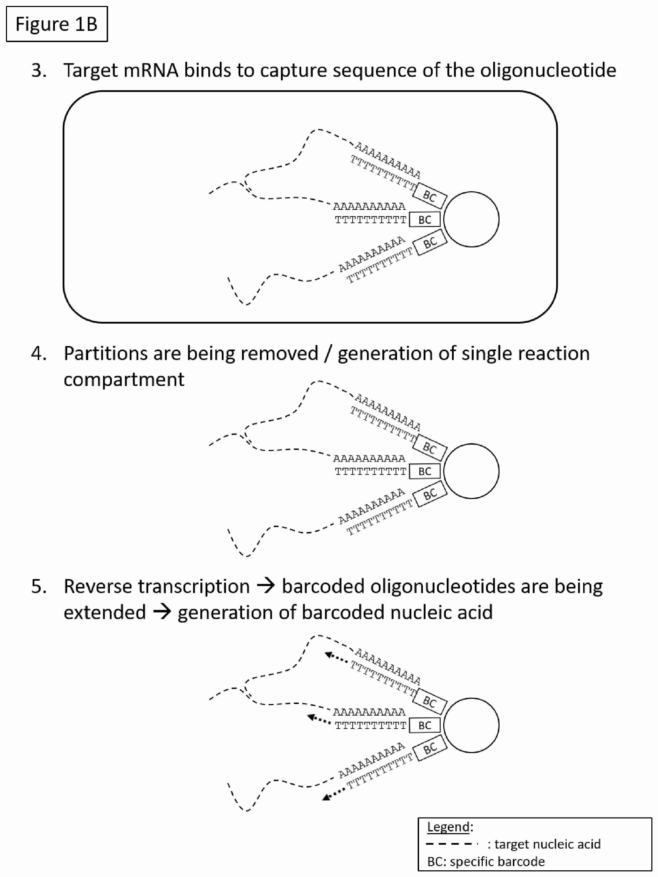

However current state in the art methods, as shown in figure 1, have certain drawbacks. When using methods utilizing droplets it is very difficult to add reagents once droplets have been formed. Therefore, all reagents for accessing the biological particle (e.g. lysis of a cell), isolation of the molecules of interest (e.g. mRNA molecules), converting the molecules of interest (e.g. cDNA synthesis for mRNA molecules), and incorporating the barcode have to be added simultaneously, and the respective reactions have to be conducted in the same reaction mixture. An example for such a workflow is the Chromium Next GEM Single Cell 5' Library & Gel Bead kit v1.1 (PN-100165, 10x Genomics, Pleasanton, CA, USA).

In contrast to that, methods utilizing small wells allow the addition of reagents during the process described in the paragraph above. However it is not possible to exchange reagent mixtures. Therefore reactants of previous steps could still impact the subsequent reactions. An example for such an workflow is the Smart-seq2 approach by Picelli et al. (2014). WO2020/112604 discloses single cell droplet based barcoding of transcripts and gDNA.

In both cases, it is impossible to conduct a complete buffer exchange between the different workflow steps, therefore the respective reactions have to be conducted under non-ideal reaction conditions.

Some methods try to circumvent these limitations by using a hybridization-based approach to isolate the molecules of interest on barcoded capture molecules, which are coupled to beads. The bound target nucleic acids can then be isolated and a barcode can be incorporated in subsequent steps using ideal reaction conditions. An example for such a workflow is the drop-seq approach by Macosko et al. (2015). However there are several limitations e.g. in the case of barcoding all mRNA molecules, this approach can only be used to add the barcode via the capture sequence (oligo(dT)), therefore the barcode will be adjacent to the 3' end of the transcript. An additional disadvantage of that method is, that it is difficult to use target specific primers which limit the reverse transcription to only a subset of mRNA or other RNA species (in contrast to all poly(A) RNA when using oligo(dT) primers), as each of the specific primer has to be linked to the barcode on the surface of the bead.

However for several downstream applications a barcode sequence at the 5' end of the transcript is essential e.g. T cell receptor sequencing using kits like the 10x Genomics Single Cell Immune Profiling kits. To achieve this, a process termed template switching is being employed (Zhu et al, 2001). The template switching process is a transient process and therefore has to be conducted while the mRNA molecules of the different cells are still partitioned. As a consequence, at least two process steps (cell lysis and reverse transcription with template switching) have to be conducted while cells are partitioned (in droplets or wells). Both reactions therefore have to be performed under non ideal conditions. Especially for template switching reactions specific conditions e.g. buffer conditions are needed in order to work properly and generate optimal results. This is one of the main limitations in the state of the art.

Based on these limitations, current state of the art workflows also fail to provide solutions for the combined analysis of different target nucleic acid molecules such as mRNA and genomic DNA from the same cell, in a single step. Such a method would provide a great improvement as less cell material would be needed on the one hand and on the other hand, it would facilitate analyzing and directly comparing mRNA and genomic DNA of the same cell.

For the given reasons performing several reactions in one partition stays a major limitation in sample preparation and nucleic acid barcoding workflows.

### Brief description of the invention

Our researchers surprisingly found a method which allows the separation of the different workflow steps for barcoding of target nucleic acids and therefore providing optimal reaction conditions for each workflow step, especially for template switching reactions. Moreover this method provides the opportunity to perform the reactions such as barcoding reactions of two different nucleic acid molecules from one cell such as RNA and genomic DNA molecules in a single step.

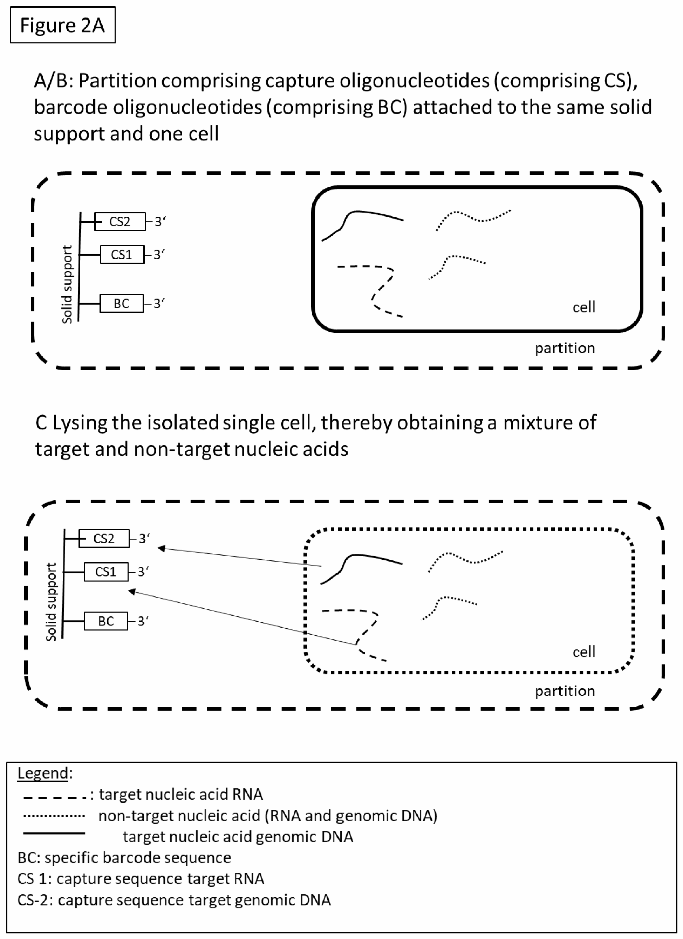

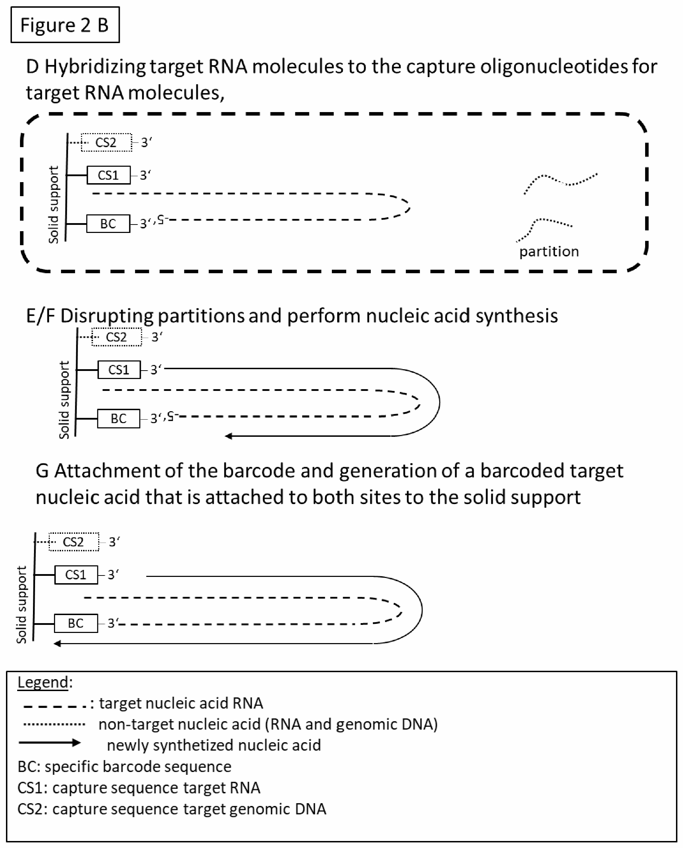

One object of the invention is to provide a method for the generation of barcoded target nucleic acids from a plurality of cells comprising the steps:
a. Providing a plurality of cells comprising target RNA molecules and at least one solid support comprising capture oligonucleotides for said target RNA molecules and barcode oligonucleotides (figure 2A part A/B)
b. Partitioning said plurality of cells and said solid supports such that each cell is included into a separate partition and each partition comprises a solid support (figure 2A part A/B)
c. Lysing said cell, thereby obtaining a mixture of target and non-target RNA molecules (figure 2A part C)
d. Hybridizing said target RNA molecules to the capture oligonucleotides for said target RNA molecules, thereby obtaining target RNA molecules attached to said solid support (figure 2B part D)
e. Disrupting the partitions and separating the non-target RNA molecules from the target RNA molecules attached to said solid support (figure 2B part E/F)
f. Generating double stranded nucleic acids from the target RNA molecules by nucleic acid synthesis which is a reverse transcription reaction with template switching, wherein the capture oligonucleotides serve as primer, the target RNA molecules serve as templates and the barcode oligonucleotides serve as template switching oligonucleotides (figure 2B part E/F)
g. Attaching the barcode oligonucleotides to the double stranded nucleic acids from target RNA molecules, thereby generating barcoded nucleic acids from target RNA molecules (figure 2B part G)
**Characterized in that** in step a) said plurality of cells additionally comprise target genomic DNA molecules and said at least one solid support additionally comprise capture oligonucleotides for target genomic DNA molecules and **in that** the capture oligonucleotides for target RNA and target genomic DNA molecules are different.

In a first variant of the invention steps a-f) are also performed for target genomic DNA molecules, thereby generating double stranded target genomic DNA molecules attached to the same solid support as the double stranded nucleic acids from target RNA molecules. Optionally step g) may be performed in order to obtain barcoded double stranded target genomic DNA molecules. The final outcome of this method are then barcoded target nucleic acids (RNA and DNA) attached to with sites to the same solid support.

In a second variant, the invention describes a method for generating barcoded target RNA molecules and simultaneously performing whole genome amplification of target genomic DNA molecules.

### Brief description of the drawings

Figure 1A/B: standard single-cell cDNA barcoding workflow: (1) In a first step, a single cell and a single bead is compartmentalized in a partition (e.g. droplet or well). The bead contains an oligonucleotide comprising an unique barcode (each oligonucleotide on the bead has the same barcode, but no other bead/ partition in this experiment will have the same barcode) and an oligo(dT) oligonucleotide (SEQ ID NO:6). (2) The cell is lysed. (3) The mRNA molecules hybridize via their polyA tail (SEQ ID NO: 5) to the oligo(dT) oligonucleotide (SEQ ID NO:6) on the bead. (4) The partitions are disrupted (e.g. in case of droplet based approaches, the emulsion is broken using chemicals). The mRNA remains bound to the bead. (5) The mRNA bound to beads is subjected to a reverse transcription reaction (in bulk). During this process, a barcoded cDNA molecule is generated by extending the barcoded oligonucleotide on the bead.
Figure 2: Principle of invented method (example mRNA analysis). step A/B) shows a Partition comprising capture oligonucleotides (for target RNA and for target genomic DNA), barcode oligonucleotides attached to the same solid support and one cell. There follows lysing the cell, thereby releasing nucleic acid molecules and obtaining a mixture of target and non-target nucleic acids (step C). The following steps of the method are depicted for target RNA only, as the steps for target genomic DNA is dependent on the specific embodiment of the invention (these embodiments are shown in the following figures). In D there follows hybridizing target nucleic acid molecules (RNA) to the capture oligonucleotides. Then partitions are disrupted and a nucleic acid synthesis is performed (E/F). Then the barcode oligonucleotide is attached to generate a barcoded target nucleic acid (G). The nucleic acid (from target RNA) is attached to both sites of the solid support.

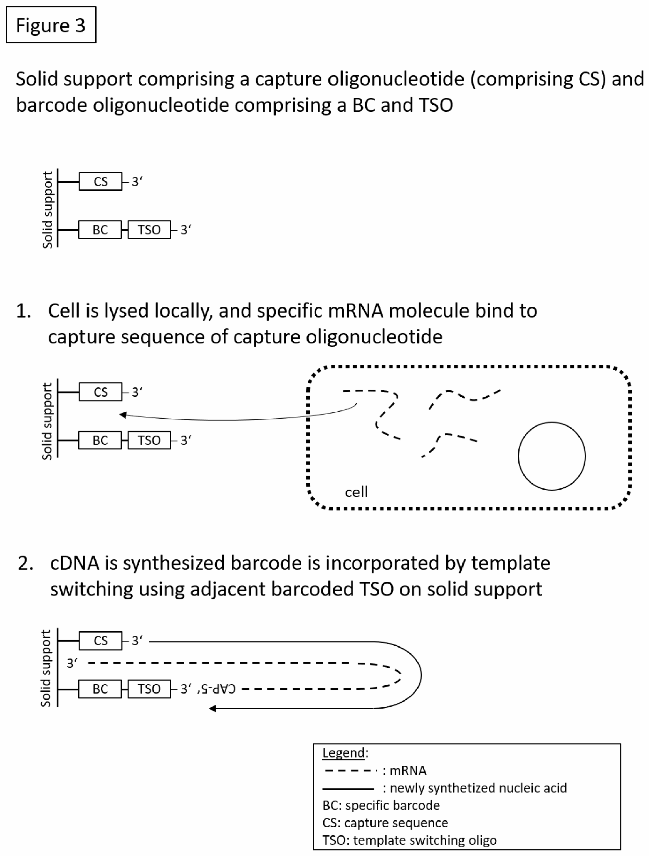

Figure 3: This figure is to illustrate the principle of target RNA barcoding by incorporation of barcode by template switching. (For sake of clarity, capture oligonucleotides for target genomic DNA are not shown/illustrated.) In this example, a solid support comprising a capture (for target RNA molecules) and barcode oligonucleotide and a template switching oligonucleotide sequence is being used. (1) The single cell is lysed locally. The target RNA molecules will bind to the specific capture oligonucleotides on the solid support (e.g. mRNA molecules binding to a capture oligonucleotide comprising a capture sequence comprising a oligo(dT) sequence). The partitions are disrupted. (2) cDNA is being synthesized. During cDNA synthesis, the template switching oligonucleotide comprised in the barcode oligonucleotide on the same solid surface is being used, thereby incorporating the barcode into the newly synthesized cDNA molecule.

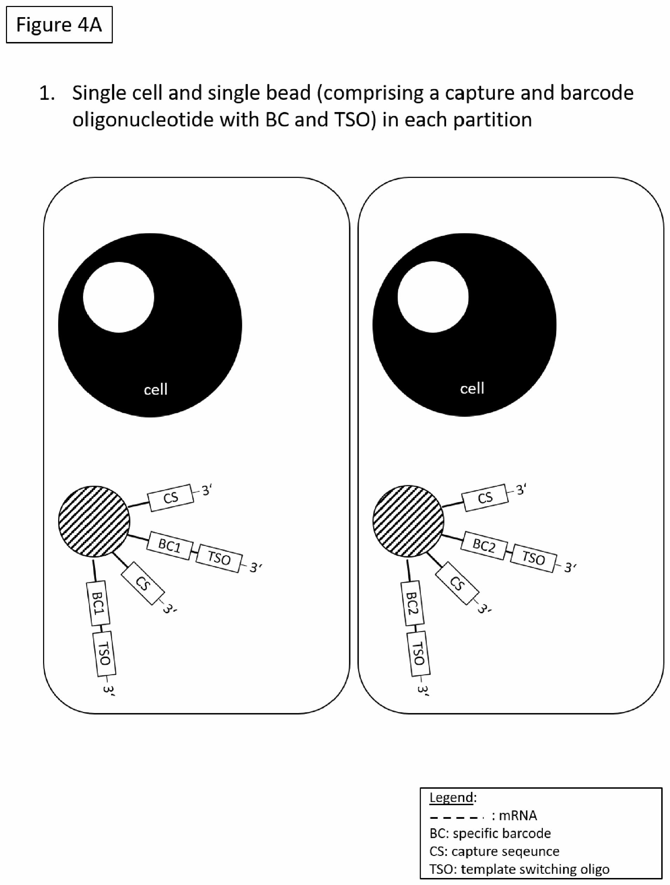

Figure 4 A-C: Example of using the invented method for barcoding cell-specific cDNA using beads as solid supports. (For sake of clarity, capture oligonucleotides for target genomic DNA are not shown/illustrated.) The example uses beads comprising a capture oligonucleotide and barcode oligonucleotide comprising a barcode oligonucleotide (comprising a barcode sequence (BC)) and a template switching oligonucleotide (TSO) sequence. (1) Single cells and single beads are partitioned, each partition containing one cell and one single bead. The example shows two different partitions with one cell and one bead each. Each bead comprises a different barcode sequence (bead-specific barcode denoted as BC1 and BC2, respectively). (2) Cells are lysed within the partition and target mRNA molecules bind to the capture oligonucleotide on the bead. The capture oligonucleotide comprises a capture sequence which may be an oligo(dT) binding poly(A) mRNA molecules or a specific sequence binding a subset of mRNA molecules.(3) All beads are combined in a single compartment, for example by breaking the emulsions in case partitioning was accomplished by water-in-oil droplets. In the single compartment, a reverse transcription reaction is being conducted using a reverse transcriptase capable of template switching. The template switching will be conducted using the template switching oligonucleotide on the same bead. Thereby, the cDNA molecules generated from the same cell will comprise the same barcode (bead-specific barcode will become cell specific barcode).

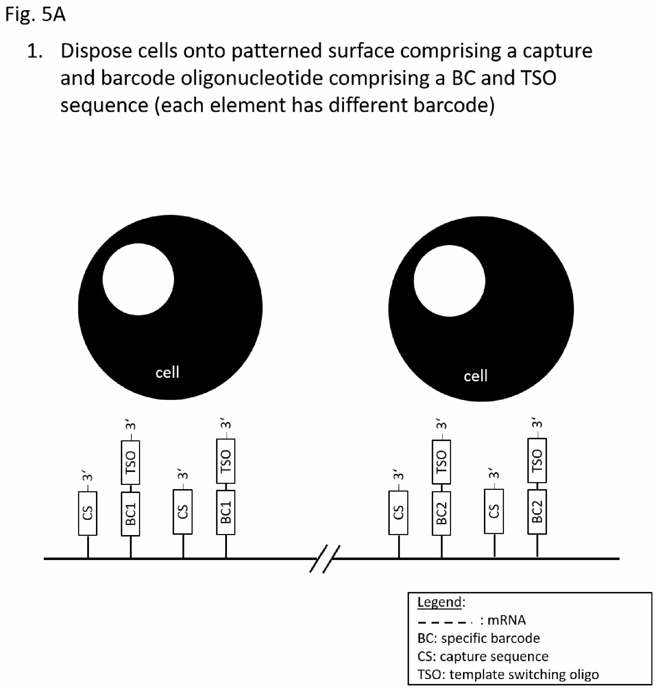

Figure 5 A-C: Example of using the invented method for barcoding cell-specific cDNA using a patterned surface as solid support. (For sake of clarity, capture oligonucleotides for target genomic DNA is not shown/illustrated.) The example uses a patterned surface comprising a capture oligonucleotide (comprising a capture sequences CS) and barcode oligonucleotide (comprising a barcode sequence (BC)) and a template switching oligonucleotide sequence (TSO). The surface contains patterns of these barcode oligonucleotides with specific barcodes, where different pattern elements of the surface contain only a single barcode. (1) Single cells are deposited on the surface. The cells are deposited in a way that each pattern area will contain no more than one cell. (2) Cells are lysed locally under conditions minimizing diffusion from one area to another. This may be achieved by specific buffer conditions, or by generating temporary barriers between the areas (e.g. by using hydrophilic surfaces and depositing cells in aqueous droplets surrounded by oil). The target mRNA molecules will bind to the capture oligonucleotide on the patterned surface. The capture oligonucleotide comprises a capture sequence which may be an oligo(dT) binding poly(A) mRNA molecules or a specific sequence binding a subset of mRNA molecules. (3) A reverse transcription reaction is being conducted using a reverse transcriptase capable of template switching. During reverse transcription and template switching, the adjacent barcode (pattern area specific barcode) is being incorporated into the cDNA. The pattern element specific barcode will become the cell specific barcode.

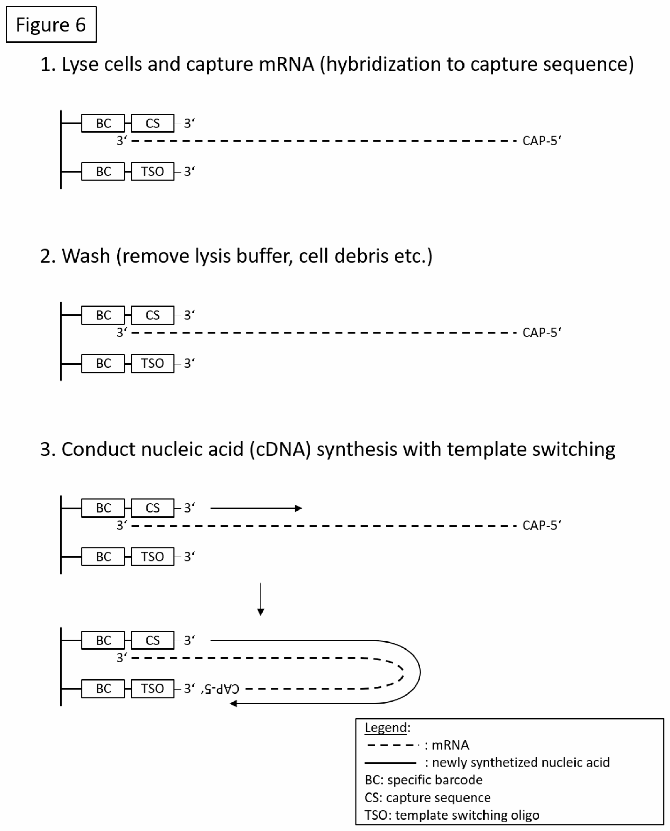

Figure 6: Example for using the invented method for dual barcoding of mRNA molecules. (For sake of clarity, capture oligonucleotides for target genomic DNA is not shown/illustrated.) The example uses a surface as solid support comprising a capture (comprising a capture and a barcode sequence) and barcode oligonucleotide comprising a barcode sequence and a template switching oligonucleotide sequence. Said solid support is partitioned with a single cell. (1) the cell is lysed and the target mRNA binds to the barcoded capture oligonucleotide. (2) The solid surface is washed in order to remove lysis buffer, cell debris and/or other substances interfering with subsequent process steps.(3) A reverse transcription reaction is being conducted using a reverse transcriptase capable of template switching reaction. The reverse transcription is primed by the barcoded capture oligonucleotide, thereby incorporating the barcode of the capture oligonucleotide into the newly synthesized cDNA. During reverse transcription and template switching, the adjacent barcode (pattern area specific barcode) is being incorporated into the cDNA. Thereby, the barcode (reverse complement) of the template switching oligonucleotide is also incorporated into the newly synthesized cDNA. The resulting cDNA will therefore contain a barcode at both ends. The barcode of the capture oligonucleotide and the template switching oligonucleotide may be identical, thereby the same barcode (same orientation or reverse complement) is incorporated into the cDNA molecule. This is of great advantage of library preparation workflows requiring fragmentation, as it allows utilizing both ends of the cDNA molecules for identification of the parental nucleic acid molecule.

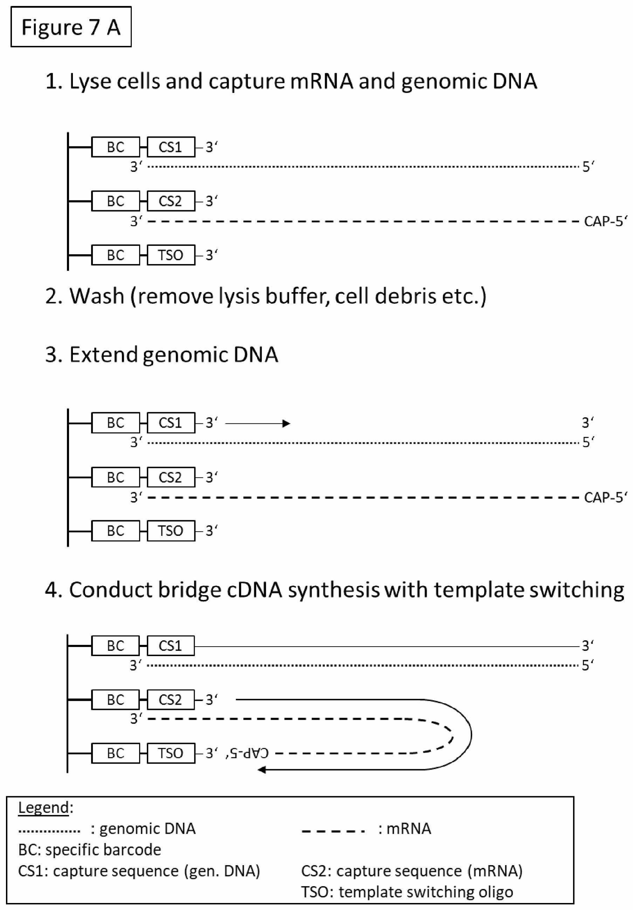

Figure 7A: Example of simultaneous barcoding of mRNA and genomic DNA molecules. The example uses a solid surface as solid support comprising a barcoded first capture oligonucleotide with a capture sequence (CS1) binding to target genomic DNA molecules, and a second barcoded capture oligonucleotide with a capture sequence (CS2) binding to target RNA molecules. In addition to that the solid support comprises a barcode oligonucleotide comprising a barcode sequence and a template switching oligonucleotide sequence (1). A single cell is partitioned to said solid support. The cell is lysed and the target genomic DNA molecules binds to the barcoded capture oligonucleotide comprising CS1, and the target RNA molecules bind to the barcoded capture oligonucleotides comprising CS2. (2) The solid surface is washed in order to remove lysis buffer, cell debris and/or other substances interfering with subsequent process steps. (3) The barcoded oligonucleotides comprising CS1 are extended with an enzyme with DNA polymerase activity using the captured target DNA molecules as template. (4) The barcoded oligonucleotides comprising CS2 are extended with an enzyme with reverse transcriptase with template switching activity using the captured target RNA molecules as template. During reverse transcription, another barcode is incorporated using the barcoded template switching oligonucleotide as template.

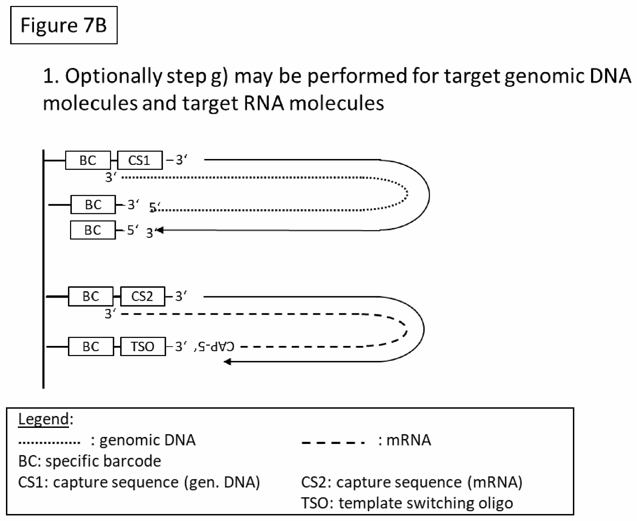

Figure 7B: optionally step G) may be performed for target genomic DNA and target RNA molecules. The barcode oligonucleotide may be attached by ligation to the target genomic DNA, whereas the barcode may be attached to the target RNA by template switching.

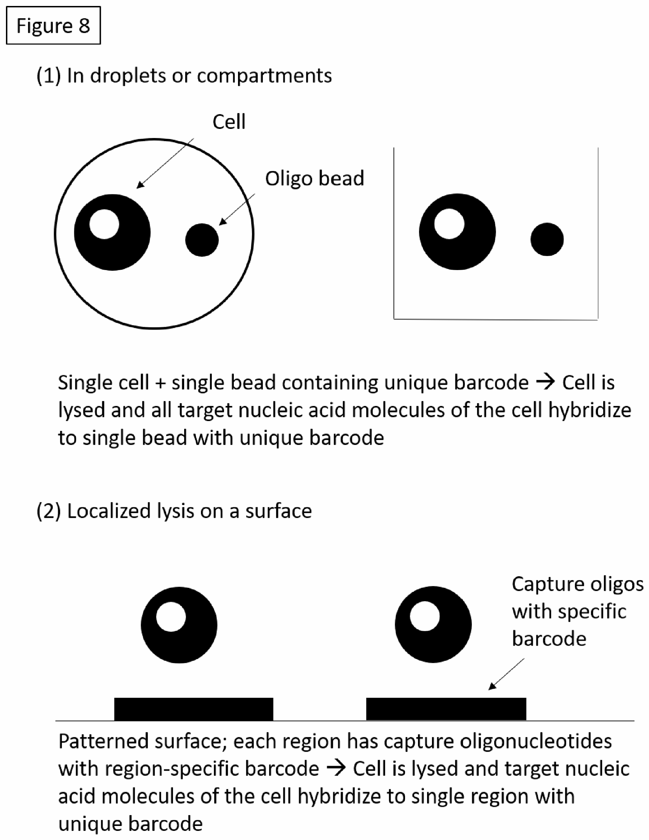

Figure 8: Examples for methods for associating single cells with a specific barcode. (1) Addition in specific compartment: a single cell and a barcode containing bead is compartmentalized in a single partition. The partition may be established via a droplet surrounded by a immiscible fluid or by wells. In the partition, cells are lysed, the nucleic acids are released and target nucleic acid molecules bind to the bead-bound capture oligonucleotides. (2) Localized lysis on a surface comprising a patterned surface with multiple areas comprising different barcodes: Cells are deposited on the surface (ideally, no more than one cell on each area comprising a specific barcode). Next, cells are lysed under conditions limiting diffusion of target molecules and target molecules bind locally. In a next step, target molecules can be converted to barcoded nucleic acids using the specific barcode of the respective area.

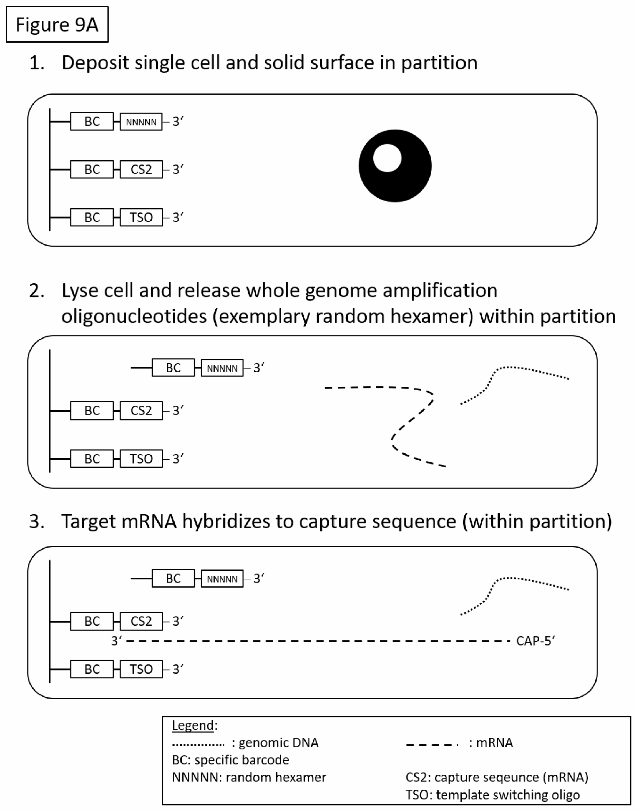

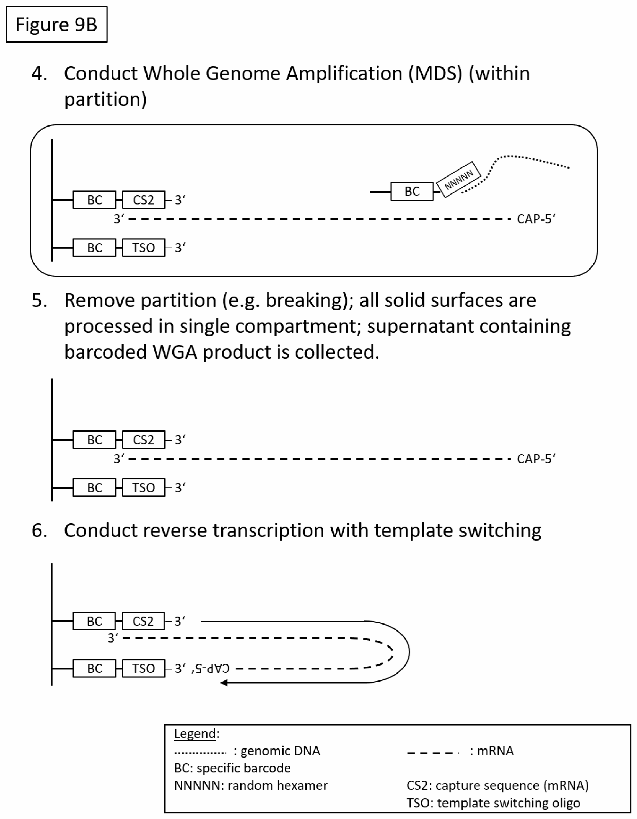

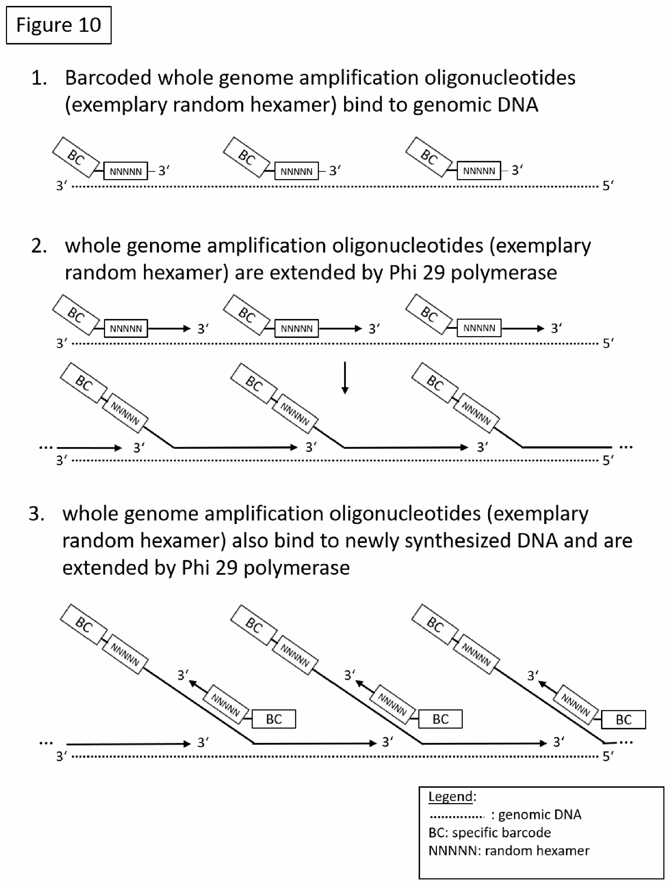

Figure 9: Example of a workflow for simultaneous barcoding of genomic DNA and RNA. In the example, solid surfaces are being used comprising (a) whole genome amplification oligonucleotide, (b) capture oligonucleotides comprising capture sequence and a barcode sequence specific for target RNA (CS2), and (c) a barcode oligonucleotide comprising template switching oligonucleotide. The solid surface contains a plurality of each type of oligonucleotide - for simplicity, only a single copy is shown in the figure. (1) A single cell is deposited in a partition comprising a solid surface with a plurality of whole genome amplification oligonucleotide (comprising a barcode), a plurality of barcoded oligonucleotides with a RNA capture sequence, and a plurality of barcode oligonucleotides comprising a template switching oligonucleotide. The barcode of the solid surface is specific for the partition. (2) In a first step, the cell in the partition is being lysed, the nucleic acid molecules are released and the whole genome amplification oligonucleotide is being released from the solid surface. The cell contains a plurality of RNA and DNA molecules - for simplicity, only a single copy is shown in the figure. (3) The target RNA hybridizes to the capture sequence of the barcoded capture oligonucleotide that is still bound by the bead. (4) The target genomic DNA is amplified by whole Genome amplification. Whole genome amplification (MDA) is being conducted within the droplet using the whole genome amplification oligonucleotide (see also Figure 10), thereby generating "free" barcoded amplified target DNA (5) The partition is removed, e.g. (in case of droplet based partitions) by removing the oil (breaking). The supernatant containing the barcoded amplified genomic DNA is retained and used for downstream applications (genomic DNA based single-cell library prep). (6) The beads are subjected to a reverse transcription reaction with template switching. In this way, a barcoded cDNA is generated (barcoded at both the 5' and the 3' end).
Figure 10: Whole genome amplification (multiple displacement amplification, MDA) using whole genome amplification oligonucleotides. First, whole genome amplification oligonucleotides bind to genomic DNA. The whole genome amplification oligonucleotides serves as primer for nucleotide synthesis by Phi 29 polymerase. Phi 29 polymerase has strand displacement activity, therefore Phi 29 will continue its elongation beyond newly synthesized nucleic strands, thereby displacing other newly synthesized nucleic acid strands. Additional whole genome amplification oligonucleotides will bind to these displaced single-stranded nucleic acid strands, and additional nucleic acid molecules are generated by Phi 29 using those displaced single-stranded nucleic acid strands. As a result, multiple copies of the genomic DNA will be synthesized, and each of these copies will have a barcode at the 5' end of the newly synthesized nucleic acids.

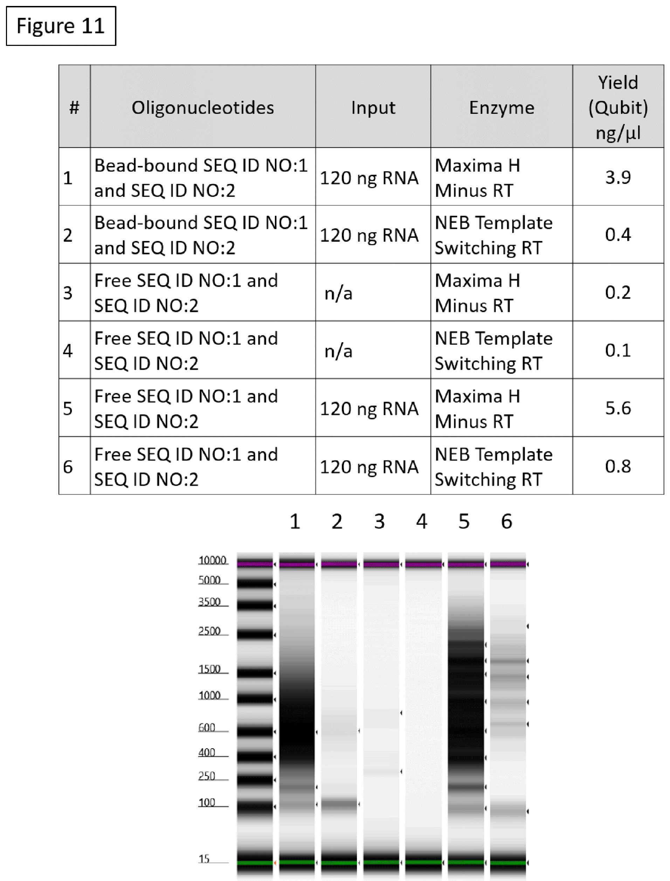

Figure 11: shows the results of example 1 (cDNA amplification on beads).

### Detailed description of the invention

In the first aspect the present invention provides a method for the generation of barcoded target nucleic acids from a plurality of cells comprising the steps:
a. Providing a plurality of cells comprising target RNA molecules and at least one solid support comprising capture oligonucleotides for said target RNA molecules and barcode oligonucleotides
b. Partitioning said plurality of cells and said solid supports such that each cell is included into a separate partition and each partition comprises a solid support
c. Lysing said cell, thereby obtaining a mixture of target and non-target RNA molecules
d. Hybridizing said target RNA molecules to the capture oligonucleotides for said target RNA molecules, thereby obtaining target RNA molecules attached to said solid support
e. Disrupting the partitions and separating the non-target RNA molecules from the target RNA molecules attached to said solid support
f. Generating double stranded nucleic acids from the target RNA molecules by nucleic acid synthesis which is a reverse transcription reaction with template switching, wherein the capture oligonucleotides serve as primer, the target RNA molecules serve as templates and the barcode oligonucleotides serve as template switching oligonucleotides
g. Attaching the barcode oligonucleotides to the double stranded nucleic acids from target RNA molecules, thereby generating barcoded nucleic acids from target RNA molecules
**Characterized in that** in step a) said plurality of cells additionally comprise target genomic DNA molecules and said at least one solid support additionally comprise capture oligonucleotides for target genomic DNA molecules and **in that** the capture oligonucleotides for target RNA and target genomic DNA molecules are different.

The invented method is for the generation of barcoded target nucleic acids from a plurality of cells. The barcoded target nucleic acids may be used for several downstream applications such as library preparation, next generation sequencing or polymerase chain reactions.

The plurality of cells may be derived from a sample and comprise single cells comprising target nucleic acids. Samples that may be used for the generation of barcoded target nucleic acids as described herein, may originate from any specimen, like whole animals, organs, tissue slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo sapiens). A biological sample may have the form of a tissues slice, cell aggregate, suspension cells, adherent cells or body fluids.

The target nucleic acid are RNA (such as mRNA) and genomic DNA molecules.

The target nucleic acids may encode for T cell receptor chains and/or B cell receptor chains. In one embodiment target RNA molecules may encode for T cell receptor chains and/or B cell receptor chains. In another embodiment target genomic DNA molecules may encode for T cell receptor chains and/or B cell receptor chains. In yet another embodiment of the invention, the target RNA and the target genomic DNA molecules may encode for T cell receptor chains and/or B cell receptor chains.

In another embodiment the target nucleic acids may encode for biomarkers suitable for the diagnosis or treatment of a disease. In one embodiment target RNA molecules may encode for biomarkers suitable for the diagnosis or treatment of a disease. In another embodiment target genomic DNA molecules may encode for biomarkers suitable for the diagnosis or treatment of a disease. In yet another embodiment of the invention, the target RNA and the target genomic DNA molecules encode for biomarkers suitable for the diagnosis or treatment of a disease.

In yet another embodiment of the invention the target nucleic acid molecules may encode for a fusion gene generated by chromosomal rearrangements. In one embodiment target RNA molecules may encode for a fusion gene generated by chromosomal rearrangements. In another embodiment target genomic DNA molecules may encode for a fusion gene generated by chromosomal rearrangements. In yet another embodiment of the invention, the target RNA and the target genomic DNA molecules encode for a fusion gene generated by chromosomal rearrangements.

In the first step (a) of the invented method a plurality of cells comprising target nucleic acids (target RNA and target genomic DNA molecules) and at least one solid support comprising capture oligonucleotides and barcode oligonucleotides are provided.

The **capture oligonucleotides** (for target RNA and target genomic DNA molecules) comprise a capture sequence (CS), which is at least partially complementary to the target nucleic acids (target RNA or target genomic DNA molecules). The capture sequence can be varied, depending on the target RNA or target genomic DNA molecules. Capture oligonucleotides are single stranded. In one embodiment the capture sequence may be an oligo dT sequence, which is capable of binding mRNA molecules (poly(A) tail). In another embodiment the capture sequence may contain a random sequence, which complementarily binds to all target nucleic acid molecules (DNA and/or RNA) species. In another embodiment the capture sequence of the capture oligonucleotide may be complementary to specific target nucleic acid molecules (DNA and/or RNA), thus enabling the binding of specific target nucleic acids.

In another embodiment the capture sequence may be complementary to a specific target sequence such as the constant region of T-Cell or B-Cell receptors, or biomarkers for the diagnosis or treatment of disease.

One partition may comprise different capture oligonucleotides having different capture sequences, in order to bind different RNA or genomic DNA molecules or both. Moreover one partition may comprise same capture oligonucleotides having same capture sequences, in order to bind same RNA or genomic DNA molecules. In one embodiment one partition may comprise (same) capture oligonucleotides for target RNA molecules in order to bind RNA molecules having a same sequence (at least partially) and the partition may additionally comprise (same) capture oligonucleotides for target genomic DNA in order to bind DNA molecules having a same sequence (at least partially). Some capture oligonucleotides may be specific for RNA and some for genomic DNA. In one embodiment the capture sequence of the capture oligonucleotides specific for RNA binding is an oligo d(T) sequence. In contrast to that, the capture sequence of the capture oligonucleotide specific for genomic DNA may be random hexamer

In another embodiment different partitions may comprise capture oligonucleotides comprising same or different capture sequences. In a preferred embodiment the capture oligonucleotides among the plurality of partitions comprise the same capture sequences.

The capture oligonucleotide may further comprise a barcode sequence (referred to as barcoded capture oligonucleotide), a primer binding sequence or both.

Such an oligonucleotide is also referred to as barcoded capture oligonucleotide. The barcode sequence may be a cellular barcode and/ or a unique molecular identifier (UMI). In one embodiment of the invention the capture oligonucleotides for target genomic DNA molecules may comprise a barcode sequence (UMI or cellular barcode). Such a barcode may be preferentially a cellular barcode. In another embodiment of the invention the capture oligonucleotides for target RNA and target genomic DNA may comprise a barcode sequence (UMI or cellular barcode). This barcode sequence may be same (cellular barcode) or different (UMI). In a preferred embodiment the barcode sequence may be a cellular barcode. In other words the barcode sequence may be same for capture oligonucleotides for target RNA and target genomic DNA.

The primer binding sequence may comprise a sequencing and/ or amplification primer binding site to enable nucleic acid amplification and/or sequencing reactions as downstream applications.

The **barcode oligonucleotides** comprise at least one barcode sequence. Said barcode sequence may be same or different within one partition. In addition to that said barcode sequence may be same or different among the plurality of partitions. In another embodiment, barcode sequences of the barcode oligonucleotides provided in step a) are different among the partitions and barcode sequences of the barcode oligonucleotides comprised in a partition are the same. Based on that barcode oligonucleotides provided in step a) may be different among the partitions and barcode oligonucleotides comprised in a partition may be the same.

The barcode sequence may be a cellular barcode and/ or a unique molecular identifier (UMI). In one partition the barcode oligonucleotides may comprise sequences encoding the same cellular barcode and a sequence encoding different UMIs. The sequence of the cellular barcode may be different among the plurality of partitions. The barcode oligonucleotide may further comprise a primer binding sequence. The primer binding sequence may comprise a sequencing and/ or amplification primer binding site to enable nucleic acid amplification and/or sequencing reactions as downstream applications.

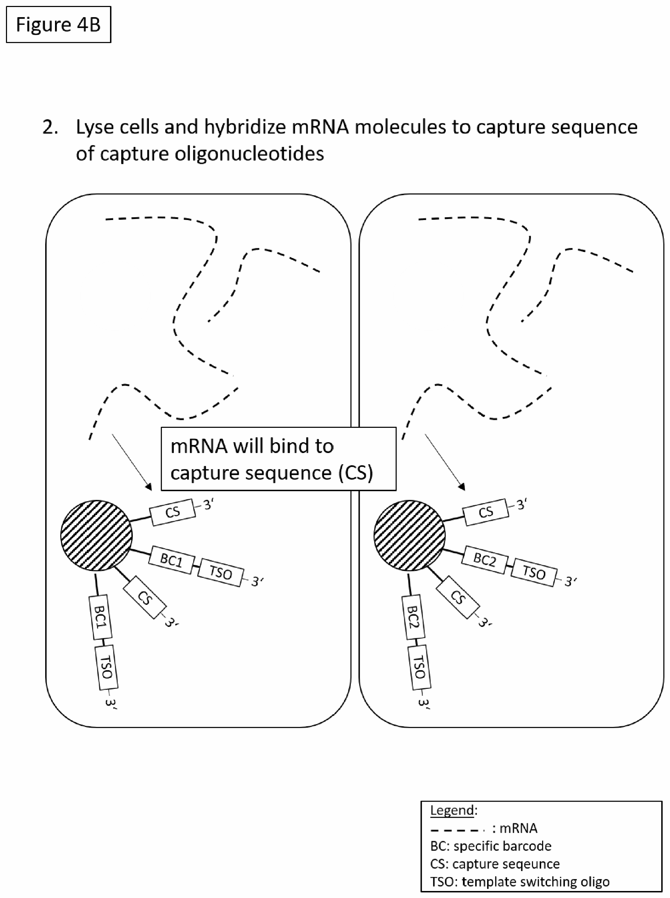

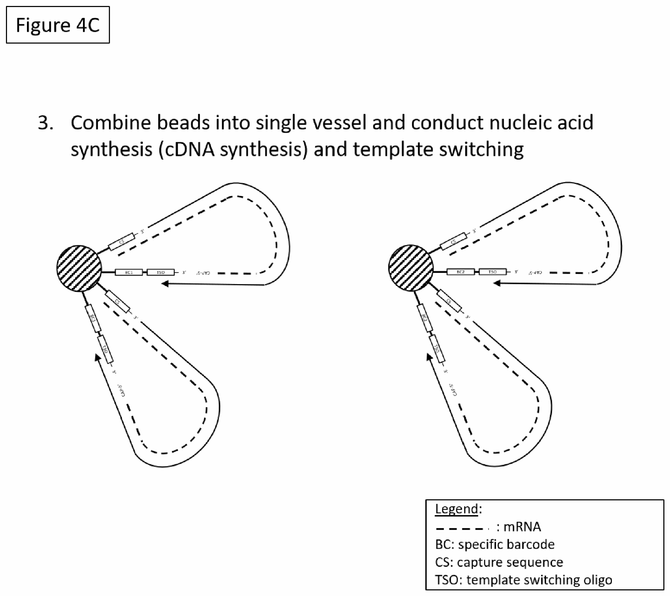

Said barcode oligonucleotide may further comprise a sequence encoding for a template switching oligonucleotide or a sequence suitable for template switching, in order to perform template switching reactions. Therefore it can be stated that the barcode oligonucleotide may serve as template switching oligonucleotide (Figure 3/4).

Said barcode oligonucleotide may be single stranded or double stranded nucleic acids. In one embodiment of the invention the target nucleic acid may be RNA and the barcode oligonucleotide may comprise a template switching oligonucleotide, wherein said barcode oligonucleotide may be single stranded.

A key feature of the invented method is the **solid support** comprising capture oligonucleotides for target RNA and target genomic DNA molecules and barcode oligonucleotides. Said capture oligonucleotides for target RNA and target genomic DNA molecules are different.

Said solid support may be a plate or a bead. In one embodiment the solid support may be a bead (figure 4). Common examples for beads are microbeads which may be color coded or magnetic or both. In a preferred embodiment the bead is color coded. In one embodiment the partition comprises at least one bead. In a preferred embodiment the partition comprises one single bead.

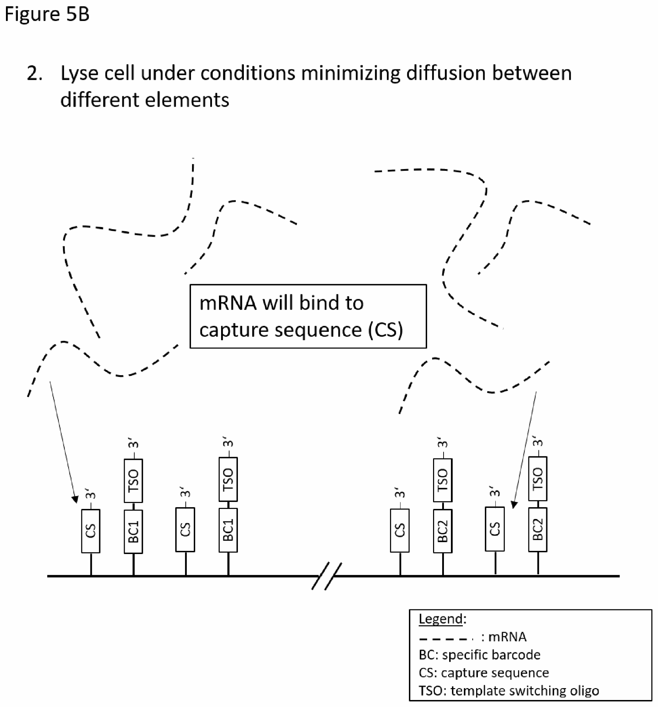

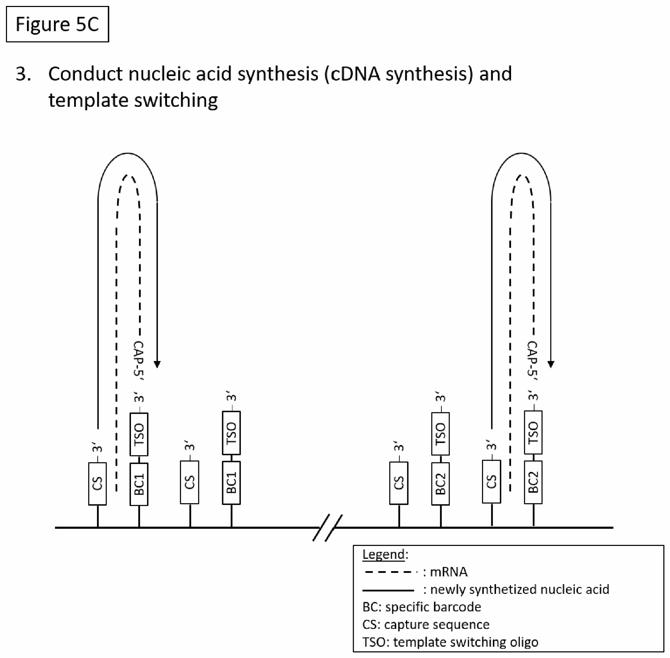

In another embodiment said solid support may be a plate (figure 5). Common examples for plates are nanoplates or microplates. The surface of such plates may be structured or patterned. The microplate may be a flat plate or a well plate, comprising a plurality of partitions. Each partition comprises a plurality of capture (for target RNA and target genomic DNA molecules) and barcode oligonucleotides. Partitioning cells according to the invention means that each partition within said plate counts as a solid support. Diffusion between partitions may be prevented by physical walls of the wells, or by chemical properties of the plate, for example by hydrophilic and hydrophobic regions. The different partitions may be sealed by a foil or another barrier. Common examples for such plates are 96- or 384-well plates, the ICELL8 nanowells (Goldstein et al. 2017), or the SCOPE-chip^{®} used by Singleron Biotechnologies (Nanjing, Jiangsu, China).

The capture (for target RNA and target genomic DNA molecules) and barcode oligonucleotides may be linked to the solid support directly (covalent) or indirectly (electrostatic interactions). Direct linkage may be achieved by linkers such as polyethylene glycol chains or other molecules such as disclosed in EP3037821A1. These linkers may have branches or multiple functional sites for increasing the number of oligonucleotides bound to the surface of a solid surface. In addition to that, the linkers may be cleavable using specific enzymatic or chemical conditions.

The link between solid support and oligonucleotide may by electrostatic interactions. Common examples are the use of biotinylated oligonucleotides binding to streptavidin on the surface of the solid support. The linkage may be released by competitive addition of biotin.

In another embodiment the oligonucleotides may contain an enzymatic cleavage site facilitating the release of the oligonucleotides from the beads.

In addition to that, other additional oligonucleotides mentioned in subsequent variants of the invention may be linked to the solid support in the same way.

In another embodiment, the solid surface may be coated with a polymer to influence diffusion behavior or movement of nucleic acid molecules bound to the capture oligonucleotides. Common examples are exemplary disclosed in US2009/0181370A1.

According to the invention a plurality of cells and said solid supports are **partitioned.** Standard procedures for partitioning are e.g. droplet formation or microwells. This may be done by standard procedures known in the art. Example protocols for partitioning cells using droplets or microwells are the Drop-seq approach (Macosco et al., 2015) or the Smart-Seq approach (Ramskold et al., 2012), respectively. In addition to that commercially available devices like the 10x Genomics Chromium Controller (10x Genomics, Pleasanton, CA, USA) can be used for partitioning of the components into droplets.

According to the invention a plurality of cells comprising target nucleic acids and at least one solid support comprising capture oligonucleotides (for target RNA and target genomic DNA molecules) and barcode oligonucleotides are partitioned. Thereby a plurality of partitions is generated. A partition comprises a cell (single cell) and a solid support, comprising capture (for target RNA and target genomic DNA molecules) and barcode oligonucleotides. The wording capture and barcode oligonucleotides can be interpreted as a plurality of capture and barcode oligonucleotides.

In one embodiment of the invention, a partition may be a droplet comprising a cell and a bead (as solid support) comprising capture and barcode oligonucleotides. Droplets may be generated according to protocols known in the art.

In another embodiment, the solid support may be a plate comprising different compartments / partitions each of which comprising capture (for target RNA and target genomic DNA molecules) and barcode oligonucleotides. Cells are deposited on the plate in a way that each partition on the plate comprises one cell.

Among the plurality of the partitions more than 1%, more than 10%, more than 50 % of the partitions have the given composition. In a preferred embodiment at least 50% of the partitions comprise a solid support and one cell.

It is understood that the partition further comprises reagents needed for the reactions of the method and its embodiments. The partition may therefore comprise a reaction buffer and enzymes such as enzymes for amplification, ligation, fragmentation, whole genome amplification.

In the next step **(step c)** of the invented method, the cell is lysed within the partition. As a consequence the nucleic acid molecules are released into the partition and a mixture comprising target and non-target nucleic acids is obtained. More specifically a mixture of target RNA and non-target RNA molecules. It is understood that the mixture further comprises target genomic DNA and non-target genomic DNA molecules.

Lysis of the cells may be done enzymatically and/or chemically using specific buffer conditions. Several methods and compositions are known in the art. An example for a lysis buffer compatible with subsequent hybridization of RNA (especially mRNA) to capture oligonucleotides is the Lysis/Binding Buffer for Dynabeads^{™} mRNA Purification Kits (Cat. No. A33562, ThermoFisher Scientific, Waltham, MA, USA).

Then, in **step d)** of the invented method, the released target RNA molecules hybridize to the capture oligonucleotides (for said target RNA molecules), thereby obtaining target RNA molecules attached to the solid support. Hybridization is achieved by complementary or partial complementary binding of the capture sequence (comprised in the capture oligonucleotide) and the complementary sequence in the target nucleic acid (here RNA). Hybridization conditions are known in the art.

Then **(step e)** the partitions are disrupted and the non-target RNA molecules are separated from target RNA molecules that are attached to the solid support. Disruption of the partitions may be done chemically using specific buffer conditions. After the disruption of the partitions the non-target nucleic acids such as non-target RNA molecules are in the supernatant in solution, while the target RNA molecules are attached to the solid support. By removing the supernatant the non-target nucleic acids such as non-target RNA molecules can be separated from the target RNA molecules. This may be done by sedimentation of the target RNA molecules attached to a solid support and removing the supernatant comprising non-target nucleic acids such as non-target RNA molecules. The supernatant may be removed by pipetting. Other methods may be e.g. the application of a magnetic field or other methods known in the art.

Optionally a washing step is performed after step e) in order remove residuals of the lysis buffer and cell debris and/or other substances interfering with subsequent process steps. Buffer conditions are commonly known in the art. In a specific embodiment such a washing step is preferred.

In **step f)** a nucleic acid synthesis is performed, wherein the capture oligonucleotides for target RNA molecules serve as a primers and the target RNA molecules serve as a templates, thereby generating double stranded nucleic acids from target RNA molecules..

Said nucleic acid synthesis is a reverse transcription reaction with template switching, wherein the barcode oligonucleotides serve as template switching oligonucleotides. The generated synthetized nucleic acid is referred to as "cDNA".

Techniques and conditions for reverse transcription reaction with template switching are well known in the art. Examples for protocols for reverse transcription with template switching can be found in Zhu et al, 2001 and Wellenreuther et al., 2004. Key elements are a specific polymerase and a template switching oligonucleotide. Commonly used polymerases are the Moloney Murine Leukemia Virus Reverse Transcriptase (MMLV-RT) and its derivates, or Thermostable group II intron reverse transcriptases (TIGRT).

It is understood that additional components may be added in order to perform such a reaction, such as buffer, nucleotides (dNTPs) and polymerase.

In **step g)** there follows a step of attaching the barcode oligonucleotides to the double stranded nucleic acids from target RNA molecules synthesized in step f), thereby generating barcoded nucleic acids from target RNA molecules. The barcoded nucleic acids from target RNA molecules comprise the capture oligonucleotides (for targe RNA molecules) and said barcode oligonucleotides, wherein the double stranded nucleic acids are attached with both ends to the same solid support. Attaching the barcode oligonucleotides may be done by ligation by ligases, template switching during the reverse transcription reaction, or other reactions well known in the art. Common examples for such ligases are e.g. T4 DNA ligase, Taq ligase or equivalent enzymes. Common examples for enzymes facilitating reverse transcription are the MMLV reverse transcriptase and its derivates.

In one embodiment the target nucleic acid molecules (e.g. from target RNA and genomic DNA) are released after step g) from the solid support. In one embodiment, the capture and barcode oligonucleotides may be linked to said solid support by a linker, wherein said linker is cleaved after step g) thereby releasing said target nucleic acid molecules from the solid support. More specifically the capture oligonucleotides for target RNA molecules, the capture oligonucleotides for target DNA molecules and the barcode oligonucleotides are linked to said solid support by a linker, wherein said linker is cleaved after step g) thereby releasing said nucleic acids from said solid support.

The release reaction may be chemically and/or enzymatically by disputing the binding between solid support and capture/ barcode oligonucleotides. Common techniques are known in the art e.g. as disclosed in EP3037821A1. One example may be the use of photocleavable linkers in the oligonucleotides coupled to the solid support. The release may also be mediated by a site specific endonucleases such as restriction enzymes. In this embodiment, the capture and barcode oligonucleotides are being cleaved after step g) by a site specific endonuclease, thereby releasing said barcoded nucleic acids from the solid support. This is based on recognition sites incorporated into the respective oligonucleotides, allowing to release the nucleic by restriction enzymes. Another example is to incorporate specific bases in the oligonucleotides that can be recognized by DNA glycosylases, for example to incorporate one or multiple uracil bases that can be excised by the USER enzyme or the thermolabile USER enzyme from New England Biolabs (Cat. No M5508 and M5507, New England Biolabs, Ipswich, MA, USA), thereby generating a nick in the respective nucleic acid strand, thus allowing the release of the barcoded target nucleic acid molecule.

The target nucleic acids can then be used for downstream applications such as nucleic acid amplification and sequencing. Based on that a gene expression analysis, mutation analysis or the analysis of copy number variations can be done.

### Target molecules - Genomic DNA

In a first variant of the invention, the steps a-f of the method described in aspect 1 is also performed for target genomic DNA molecules (Figure 7). It is understood that all embodiments described in the first aspect of the invention also apply for this variant of the invention.

In this variant of the invention the mixture obtained in step c) additionally comprises target genomic DNA and non-target genomic DNA molecules. Consequently the complete mixture which is obtained in step c) comprises target genomic DNA and non-target genomic DNA molecules, target RNA and non-target RNA molecules.

Moreover in step d) said target genomic DNA molecules are hybridized to the capture oligonucleotides for target genomic DNA molecules, thereby obtaining target genomic DNA molecules attached to said solid support. Consequently the final outcome of step d) is target genomic DNA molecules and target RNA molecules, which are attached to the same solid support.

In addition to that in step e) additionally the non-target genomic DNA molecules are separated from the target genomic DNA molecules attached to said solid support. Consequently, in step e) of this variant of the invention the non-target genomic DNA molecules and the non-target RNA molecules are separated from the target genomic DNA and target RNA molecules which are attached to the same solid support.

Finally in step f) double stranded nucleic acids from the target genomic DNA molecules are generated by nucleic acid synthesis, wherein the capture oligonucleotides for genomic DNA molecules serve as primer and the target genomic DNA molecules serve as templates. Consequently the final outcome of step f) is double stranded target RNA and double stranded target genomic DNA attached to the same solid support.

In one embodiment of this variant of the invention step g) is additionally performed for the target genomic DNA molecules by attaching the barcode oligonucleotides to the double stranded nucleic acids from target genomic DNA molecules, thereby generating barcoded nucleic acids from target genomic DNA molecules. Consequently the final outcome of this embodiment is barcoded target RNA and barcoded genomic DNA molecules.

The barcode oligonucleotides may be attached in step (g) by ligation. Ligation may be done according to methods known in the art and as disclosed herein. For optimal results double stranded nucleic acid from target genomic DNA molecules obtained in step f) may be ligated to a double stranded barcode oligonucleotide. However also ligation of the double stranded nucleic acids from target genomic DNA obtained in step f) to a single stranded barcode oligonucleotide can be done.

In one embodiment of the invention the genomic DNA molecules may be fragmented and dissociated into single strands prior to hybridization (step d). Several techniques for DNA fragmentation are known in the art. DNA fragmentation may be done enzymatically. Commonly used enzymes for fragmentation are restriction endonucleases or unspecific endonucleases. Enzymatic fragmentation may also be mediated by CRISPR/Cas9. Non-enzymatic approaches like fragmentation by sonication may also be employed. The preferred length of the genomic DNA fragments is 50- 10,000 nucleotides, 75-1000 nucleotides or 100 to 250 nucleotides. In a preferred embodiment the length of the genomic DNA fragments is 50-10,000 nucleotides. In another embodiment, at least 80%, 50% or 10% of the genomic DNA molecules are be fragmented.

### Target molecules mRNA and genomic DNA and whole genome amplification

This second variant of the invention enables the combined barcoding of target RNA and genomic DNA molecules (figure 9, 10). It is understood that all embodiments described in the previous sections also apply for this variant. In this variant of the invention the capture oligonucleotides for target genomic DNA molecules serve as whole genome amplification oligonucleotides and are attached to the solid support by a releasable linker. Therefore it can be be released from the solid support within the partition. There follows a whole genome amplification of target genomic DNA comprising the steps: Releasing whole genome amplification oligonucleotides from said solid support; Performing whole genome amplification using a polymerase with strand displacement activity, wherein said whole genome amplification oligonucleotides serve as primers, thereby obtaining amplified target genomic DNA fragments.

The whole genome amplification oligonucleotides comprise at least one barcode sequences and a sequence, which is a whole genome amplification primer sequence. Based on that the whole genome amplification oligonucleotides serve as primer for whole genome amplification. Whole genome amplification primer sequence may be random hexamer sequences, degenerated primers binding to only a subset of the whole genome, or even specific primer sequences. In one embodiment the whole genome amplification primer sequence within the partition may be different. In a specific embodiment whole genome amplification primer sequences are random hexamer sequences.

The barcode sequence of the whole genome amplification oligonucleotides may be a cellular barcode and/ or a unique molecular identifier. In one partition the barcode whole genome amplification oligonucleotides may comprise sequences encoding the same cellular barcode and a sequence encoding different UMIs. In one embodiment the whole genome amplification oligonucleotides comprises the same cellular barcode as the barcode and/or capture oligonucleotide and different UMIs

Additionally, the whole genome amplification oligonucleotides may further comprise at least one primer binding sequence. The primer binding sequence may comprise a sequencing and/ or amplification primer binding site to enable nucleic acid amplification and/or sequencing reactions as downstream applications.

Said whole genome amplification oligonucleotides are attached to the same solid support by a releasable linker. In this variant of the invention the release mechanism for the whole genome amplification oligonucleotides (capture oligonucleotides for target genomic DNA molecules) and the capture oligonucleotides for target RNA/barcode oligonucleotides is different. The release of whole genome amplification oligonucleotides (capture oligonucleotides for target genomic DNA molecules)) may be chemically and/or enzymatically by disputing the binding between solid support and said oligonucleotides. Common techniques are known in the art e.g. as disclosed in EP3037821A1. In one embodiment the whole genome amplification oligonucleotides are linked via a photocleavable linker to the solid support, and the linker may be cleaved with light. In one embodiment the linker contains a disulfide bond, and the linker may be cleaved with a reducing agent. In one embodiment the linker contains a restriction enzyme binding site, and the linker is cleaved by a specific endonuclease. In one embodiment, the linker contains an uracil base, and the linker is cleaved by a Uracil-N-Glycosylase and a endonuclease. In one embodiment, the linker contains a 8-oxo-G base, and the linker is cleaved by formamidopyrimidine [fapy]-DNA glycosylase (Fpg).

In one embodiment the whole genome amplification oligonucleotides (capture oligonucleotides for target genomic DNA molecules) and the capture oligonucleotides for target RNA/barcode oligonucleotides may be attached to said solid support by a linker, wherein the cleavable linker of said whole genome amplification oligonucleotides (capture oligonucleotides for target genomic DNA molecules) and the capture oligonucleotides for target RNA/barcode oligonucleotides are different. In one embodiment the whole genome amplification oligonucleotides (capture oligonucleotides for target genomic DNA molecules) and the capture oligonucleotides for target RNA/barcode oligonucleotides may comprise a enzymatic cleavage sequence, which is different. In yet another embodiment the whole genome amplification oligonucleotides whole genome amplification oligonucleotides (capture oligonucleotides for target genomic DNA molecules) may comprise an enzymatic cleavage sequence, while the the capture oligonucleotides for target RNA/barcode oligonucleotides may be linked to the solid support by a linker, which can be cleaved or vice vera. Thus enabling specific release of the whole genome amplification oligonucleotides (capture oligonucleotides for target genomic DNA molecules).

In this method barcoded RNA and genomic DNA molecules are generated. Based on that two different reactions are performed within the partition:

(1) The target RNA binds complementary to said capture sequence of the capture oligonucleotide for target RNA molecules (figure 9A; 2). There follows a reverse transcription reaction with template switching in step f), wherein the barcode oligonucleotides serve as template switching oligonucleotides (figure 9B; 5/6).

(2) The target genomic DNA molecules will be barcoded by whole genome amplification (after step b) by: Releasing the whole genome amplification oligonucleotides (capture oligonucleotides for target genomic DNA molecules) from said solid support (figure 9A; 2). Then a whole genome amplification is performed within the partition, wherein the released whole genome amplification oligonucleotides (capture oligonucleotides for target genomic DNA molecules) serve as primers and wherein the polymerase is a polymerase with strand displacement activity. The amplified target genomic DNA fragments are not attached to the solid support (figure 9B; 4).

Based on that in step e) a mixture is obtained comprising: target RNA molecules attached to the solid support (RNA), target genomic DNA molecules not bound to a solid support and non-target nucleic acid molecules (non target RNA and non target genomic DNA molecules). The amplified genomic DNA molecules can be separated from the RNA bound to the solid support in step e). After the disruption of the partitions the target genomic DNA molecules are in the supernatant and can be removed. They can then be used for further workflow steps, for example for targeted amplification using a target specific primer and a primer specific to the primer binding site in the barcoded oligonucleotide..

Several whole genome amplification methods are known in the art including Multiple Displacement Amplification (MDA), Degenerate Oligonucleotide PCR (DOP-PCR) and Primer Extension Preamplification (PEP). As a result, amplified target genomic DNA molecules are obtained comprising said whole genome amplification oligonucleotides.

Whole genome amplification may be performed using a polymerase with strand displacement activity. Common examples are Phi29 polymerase, Bst DNA polymerase (large fragment), T4 DNA polymerase and T7 DNA polymerase. The preferred length of the amplified genomic DNA fragments is 50- 10,000 nucleotides, 75-1000 nucleotides or 100 to 250 nucleotides.

It is understood that additionally to the polymerase standard amplification components are required such as dNTPs, buffer conditions.

All definitions, characteristics and embodiments defined herein with regard to the first aspect of the invention as disclosed herein also apply mutatis mutandis in the context of the other aspects of the invention as disclosed herein.

### Definitions

Unless defined otherwise, technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

As used herein, the term partitioning means dividing a sample or a volume into two or more samples or volumes.

The words "binding" and "hybridize" and its grammatical exuviates may be used interchangeably. Hybridization of two nucleic acid strands occurs if they are complementary to each other. Hybridization may occur under conditions known in the art.

As used herein, the term "complementary" refers to the capacity for precise pairing between two nucleotides via Watson and crick base pairing. In explanation, if a nucleotide at a given position of a nucleic acid strand is capable of forming hydrogen bonds with a nucleotide of another nucleic acid strand, then the two nucleic acids are considered to be complementary to one another at that position. Complementarity between two single-stranded nucleic acid molecules may be "partial," in which only some of the nucleotides bind, or it may be complete when total complementarity exists between the single- stranded molecules.

A "primer" as used herein is a single stranded oligonucleotide made of nucleotides, which is able to bind to complementary nucleic acid sequences. It is understood that all primers described in the current invention may serve as starting point for nucleic acid synthesis / amplification.

The terms "nucleic acid synthesis" and "nucleic acid amplification" as used herein, can be used interchangeably. The process of nucleic acid synthesis is well known in the art. In Brief: For nucleic acid synthesis a template nucleic acid is provided, which may be single stranded or double stranded. In case double stranded nucleic acid are used initially a first step is the denaturation into single nucleic acid strands (complement and reverse complement) using techniques known in the art. No denaturation stp is needed for single stranded nucleic acids. In the next step primer are provided that bind to complementary regions of the nucleic acid strands. The 3'end of the primer is then elongated using a polymerase and a complementary strand is generated by filling with complementary nucleotides. As a result a complementary nucleic acid strand is formed. The outcome of a nucleic acid synthesis reaction is a double stranded nucleic acid.

The term "reverse transcription with template switching" is well known in the art. In brief: First, a primer is hybridized to a RNA molecule. This primer serves as priming site for the synthesis of cDNA by an enzyme with reverse transcriptase activity. Once the enzyme reaches the 5' end of the RNA template, a subset of reverse transcriptase (such as the MMLV reverse transcriptase) is capable of adding one or more additional nucleotides to the 3' end of the newly synthesized cDNA (mostly deoxycytidines). This allows the template switching oligonucleotide to bind to these deoxycytidines. Subsequently, the reverse transcriptase can switch the template and continue cDNA synthesis. Examples for protocols using reverse transcription and template switching can be found in in Zhu et al, 2001 and Wellenreuther et al., 2004.

The process of "whole genome amplification" is well known in the art. Several methods exist for whole genome amplification in the art including Multiple Displacement Amplification (MDA), Degenerate Oligonucleotide PCR (DOP-PCR) and Primer Extension Preamplification (PEP). In brief, all methods have in common that random or degenerative oligonucleotides are used for priming the nucleotide synthesis. However, it is also possible to use specific primers for only amplifying specific targets in the genome.

The term "oligonucleotide"; "nucleic acid(s)" and "nucleic acid molecules" refer to biopolymers composed of nucleotide monomers covalently bonded in a chain. An amplified nucleic acid may be named as "amplicon". A nucleic acid may be DNA or RNA.

Oligonucleotides according to the current invention may comprise a barcode sequence. A barcode sequence is a short nucleotide sequence for identification purposes. A barcode may be a cellular barcode. Here all target nucleic acids from the same cell are labeled with the same cellular barcode. In addition to that a barcode may be a unique molecular identifier (UMI), labeling all nucleic acids with different barcode sequences. A barcode sequence may comprise both a cellular barcode and a UMI.

"Random hexamer" sequences may contain all four nucleotides (A, T, G, C) in each position, or only a subset of the four nucleotides in each position. Alternative nucleotides like Uracil, or nucleotide analogues or derivates may also be used. The random hexamer oligonucleotide may also comprise a modified backbone to modify the binding behavior like locked nucleic acids (LNA) or minor grove binding (MGB) nucleotides. Instead of random hexamer sequences, random or semi-random sequences of different length may also be used.

The term "a plurality" of something as used herein means two or more.

### Examples

The following examples are intended for a more detailed explanation of the invention but without restricting the invention to these examples.

### Example 1: Bridge cDNA synthesis on solid surface:

Template switching is a common approach for introducing a specific sequence on the 5' end of newly synthesized cDNA (Zhu et al, 2001): To initiate reverse transcription using mRNA as template, a specific oligonucleotide is incubated with mRNA in the presence of a reverse transcriptase. This oligonucleotide could either contain a stretch of multiple T nucleotides to bind to the poly(A) tail of mRNA, or could be specific (reverse complement) to a single mRNA sequence. Once the reverse transcriptase reaches the 5' end of the template mRNA, certain reverse transcriptases are capable of switching template, thereby incorporating nucleotides reverse complimentary to the template switching oligonucleotide as disclosed by Zhu et al, 2001.

Current art protocols provide both the oligonucleotide for initiating the reverse transcription, as well as the template switching oligonucleotide in solution (for example the SMARTer^{®} Stranded RNA-Seq Kit, Cat. No. 634839, Takara). Even single-cell workflows like the Chromium Next GEM Single Cell 5' Library & Gel Bead kit v1.1 (PN-100165, 10x Genomics, Pleasanton, CA, USA) use both in solution (the bead bound template switching oligonucleotide is release by dissolving the gel bead). It has even been shown that releasing the barcode containing oligonucleotide delivered via beads is required for efficient cDNA synthesis in current art single cell workflows delivering barcoded oligonucleotide via a bead (Klein et al., 2015).

We therefore evaluated whether we can overcome this limitation by coupling both oligonucleotides: oligonucleotide to prime the reverse transcription reaction (according to the invention: capture oligonucleotide) and oligonucleotide for template switching (according to the invention barcode oligonucleotide comprising a template switching oligonucleotide) to a solid surface.

As a model system we used beads of approximately 1 µm size and coupled both oligonucleotides onto its surface. When kept in solution, the distance between two beads is larger than the length of a mRNA molecule. Therefore, the vast majority of template switching will only occur with primers on the same surface.

The oligonucleotide beads were generated by synthesizing the following oligonucleotides having SEQ ID NO: 1 and SEQ ID NO: 2. The oligonucleotides were biotinylated on the 5'end.

These oligonucleotides to streptavidin coated beads (Dynabeads^{™} MyOne^{™} Streptavidin C1, Thermo Fisher Scientific, Waltham, MA, USA) using the manufacturer's instructions. Note: the three last nucleotides of SEQ02 have an RNA backbone.

We next used these beads together with controls (see Figure 4) in a reverse transcription reaction using two different commercially available reverse transcriptase (Maxima RT, Cat. No. EP0742, ThermoFisher, Waltham, MA, USA and NEB template switching RT Enzyme Mix, Cat. No. M0466S, New England Biolabs, Ipswich, MA, USA).

The reverse transcription was conducted with a custom RT buffer, and the reaction was incubated at 53°C for 45 minutes. After reverse transcription, the newly synthesized cDNA was amplified for 18 cycles using the Q5 High-Fidelity polymerase (Cat. No. M0492, New England Biolabs, Ipswich, MA, USA; amplification primers: SEQ ID NO:3 and SEQ ID NO:4 ).

The yield of the obtained amplified cDNA was evaluated using the ThermoFisher Qubit 4 Fluorometer (ThermoFisher, Waltham, MA, USA), and the size distribution was assessed using an Agilent 4200 TapeStation System using D5000 or High Sensitivity D5000 Screen Tapes (Cat. No. 5067-5588 and 5067-5592, Agilent, Santa Clara, CA, USA).

The results are shown in Figure 11: The condition 1 using bead-bound oligo(dT) primer (according to the invention: capture oligonucleotide) and bead-bound template switching oligonucleotide (according to the invention barcode oligonucleotide comprising a template switching oligonucleotide ) surprisingly showed similar yields compared to the control (condition 5) using both oligonucleotides in solution, strongly indicating that "bridge cDNA synthesis" is in possible when using suitable buffer conditions.

### References

Goldstein, L.D., Chen, YJ.J., Dunne, J. et al. Massively parallel nanowell-based single-cell gene expression profiling. BMC Genomics 18, 519 (2017). https://doi.org/10.1186/s12864-017-3893-1
Klein AM, Mazutis L, Akartuna I, Tallapragada N, Veres A, Li V, Peshkin L, Weitz DA, Kirschner MW. Droplet barcoding for single-cell transcriptomics applied to embryonic stem cells. Cell. 2015 May 21;161(5):1187-1201. doi: 10.1016/j.cell.2015.04.044. PMID: 26000487; PMCID: PMC4441768.
Macosko EZ, Basu A, Satija R, Nemesh J, Shekhar K, Goldman M, Tirosh I, Bialas AR, Kamitaki N, Martersteck EM, Trombetta JJ, Weitz DA, Sanes JR, Shalek AK, Regev A, McCarroll SA. Highly Parallel Genome-wide Expression Profiling of Individual Cells Using Nanoliter Droplets. Cell. 2015 May 21; 161(5): 1202-1214. doi: 10.1016/j.cell.2015.05.002. PMID: 26000488; PMCID: PMC4481139.
Ramsköld D, Luo S, Wang YC, Li R, Deng Q, Faridani OR, Daniels GA, Khrebtukova I, Loring JF, Laurent LC, Schroth GP, Sandberg R. Full-length mRNA-Seq from single-cell levels of RNA and individual circulating tumor cells. Nat Biotechnol. 2012 Aug;30(8):777-82. doi: 10.1038/nbt.2282. Erratum in: Nat Biotechnol. 2020 Mar;38(3):374. PMID: 22820318; PMCID: PMC3467340.
Wellenreuther R, Schupp I, Poustka A, Wiemann S; German cDNA Consortium. SMART amplification combined with cDNA size fractionation in order to obtain large full-length clones. BMC Genomics. 2004 Jun 15;5(1):36. doi: 10.1186/1471-2164-5-36. PMID: 15198809; PMCID: PMC436056.
Zhu YY, Machleder EM, Chenchik A, Li R, Siebert PD. Reverse transcriptase template switching: a SMART approach for full-length cDNA library construction. Biotechniques. 2001 Apr;30(4):892-7. doi: 10.2144/01304pf02. PMID: 11314272.

## Claims

1. A method for the generation of barcoded target nucleic acids from a plurality of cells comprising the steps:
a. Providing a plurality of cells comprising target RNA molecules and at least one solid support comprising capture oligonucleotides for said target RNA molecules and barcode oligonucleotides
b. Partitioning said plurality of cells and said solid supports such that each cell is included into a separate partition and each partition comprises a solid support
c. Lysing said cell, thereby obtaining a mixture of target and non-target RNA molecules
d. Hybridizing said target RNA molecules to the capture oligonucleotides for said target RNA molecules, thereby obtaining target RNA molecules attached to said solid support
e. Disrupting the partitions and separating the non-target RNA molecules from the target RNA molecules attached to said solid support
f. Generating double stranded nucleic acids from the target RNA molecules by nucleic acid synthesis which is a reverse transcription reaction with template switching, wherein the capture oligonucleotides serve as primer, the target RNA molecules serve as templates and the barcode oligonucleotides serve as template switching oligonucleotides
g. Attaching the barcode oligonucleotides to the double stranded nucleic acids from target RNA molecules, thereby generating barcoded nucleic acids from target RNA molecules
**Characterized in that** in step a) said plurality of cells additionally comprise target genomic DNA molecules and said at least one solid support additionally comprise capture oligonucleotides for target genomic DNA molecules and **in that** the capture oligonucleotides for target RNA and target genomic DNA molecules are different.

2. A method according to claim 1, **characterized in that** capture oligonucleotides for target genomic DNA molecules comprise a barcode sequence.

3. A method according to any of the claims 1 - 2 **characterized in that** additionally steps a-f are performed for target genomic DNA molecules, wherein:
• the mixture obtained in step c) additionally comprises target genomic DNA and non-target genomic DNA molecules
• in step d) said target genomic DNA molecules are hybridized to the capture oligonucleotides for target genomic DNA molecules, thereby obtaining target genomic DNA molecules attached to said solid support
• in step e) additionally the non-target genomic DNA molecules are separated from the target genomic DNA molecules attached to said solid support
• in step f) double stranded nucleic acids from the target genomic DNA molecules are generated by nucleic acid synthesis, wherein the capture oligonucleotides for genomic DNA molecules serve as primer and the target genomic DNA molecules serve as templates

4. A method according to claim 3, **characterized in that** the target genomic DNA molecules were fragmented and dissociated into single strands prior to hybridization (step d)

5. A method according to claim 4, **characterized in that** the fragmented genomic DNA molecules have a length of 50-10 000 nucleotides

6. A method according to claim 3 -5, **characterized in that** step g) is performed additionally for target genomic DNA molecules by
G) Attaching the barcode oligonucleotides to the double stranded nucleic acids from target genomic DNA molecules, thereby generating barcoded nucleic acids from target genomic DNA molecules

7. A method according to claim 6, **characterized in that** for target genomic DNA molecules the barcode oligonucleotides are attached in step (g) by ligation.

8. A method according to any of the claims 1-7, **characterized in that** the capture oligonucleotides for target RNA molecules, the capture oligonucleotides for target DNA molecules and the barcode oligonucleotides are linked to said solid support by a linker, wherein said linker is cleaved after step g) thereby releasing said nucleic acids from said solid support.

9. A method according to claim 10, **characterized in that** the linker is cleaved enzymatically

10. A method according to any of the claims 8-9, **characterized in that** the capture oligonucleotides for target RNA molecules, the capture oligonucleotides for target DNA molecules and the barcode oligonucleotides are cleaved after step g) by a site specific endonuclease, thereby releasing said nucleic acids from said solid support

11. A method according to any of the claims 1 -2, **characterized in that** the capture oligonucleotides for target genomic DNA molecules serve as whole genome amplification oligonucleotides and are attached to the solid support by a releasable linker and
**In that** after step c) a whole genome amplification of target genomic DNA molecules is performed comprising the steps:
a. Releasing whole genome amplification oligonucleotides from said solid support
b. Performing whole genome amplification using a polymerase with strand displacement activity, wherein said whole genome amplification oligonucleotides serve as primers, thereby obtaining amplified target genomic DNA fragments

12. A method according to any of the claims claim 1-11, **characterized in that** the target nucleic acids are selected of a group consisting of nucleic acids encoding for: T cell receptor chains or B cell receptor chains

13. A method according to any of the claims 1-12, **characterized in that** at least 50% of the partitions comprise a solid support and one cell

## Patentansprüche

1. Verfahren zur Erzeugung von barcodierten Zielnukleinsäuren aus einer Vielzahl von Zellen, umfassend die folgenden Schritte:
a. Bereitstellen einer Vielzahl von Zellen, die Ziel-RNA-Moleküle umfassen, sowie mindestens eines festen Trägers, der Capture-Oligonukleotide für diese Ziel-RNA-Moleküle und Barcode-Oligonukleotide umfasst,
b. Partitionieren der Vielzahl von Zellen und der festen Träger derart, dass jede Zelle in eine separate Partition aufgenommen wird und jede Partition einen festen Träger umfasst,
c. Lysieren der Zelle, wodurch ein Gemisch aus Ziel- und Nicht-Ziel-RNA-Molekülen erhalten wird
d. Hybridisieren der Ziel-RNA-Moleküle mit den Capture-Oligonukleotiden für die Ziel-RNA-Moleküle, wodurch Ziel-RNA-Moleküle erhalten werden, die an den festen Träger gebunden sind,
e. Auflösen der Partitionen und Abtrennen der Nicht-Ziel-RNA-Moleküle von den an den festen Träger gebundenen Ziel-RNA-Molekülen
f. Erzeugen doppelsträngiger Nukleinsäuren aus den Ziel-RNA-Molekülen durch Nukleinsäuresynthese, bei der es sich um eine reverse Transkriptionsreaktion mit Template-Switching handelt, wobei die Capture-Oligonukleotide als Primer, die Ziel-RNA-Moleküle als Templates und die Barcode-Oligonukleotide als Template-Switching-Oligonukleotide dienen,
g. Anfügen der Barcode-Oligonukleotide an die doppelsträngigen Nukleinsäuren aus den Ziel-RNA-Molekülen, wodurch barcodierte Nukleinsäuren aus den Ziel-RNA-Molekülen erzeugt werden,
**dadurch gekennzeichnet, dass** in Schritt a) die Vielzahl von Zellen zusätzlich Ziel-Genom-DNA-Moleküle umfasst und der mindestens eine feste Träger zusätzlich Capture-Oligonukleotide für Ziel-Genom-DNA-Moleküle umfasst, und dass die Capture-Oligonukleotide für Ziel-RNA- und Ziel-Genom-DNA-Moleküle unterschiedlich sind.

2. Verfahren nach Anspruch 1, **dadurch gekennzeichnet, dass** die Capture-Oligonukleotide für Ziel-Genom-DNA-Moleküle eine Barcode-Sequenz umfassen.

3. Verfahren nach einem der Ansprüche 1 bis 2, **dadurch gekennzeichnet, dass** zusätzlich die Schritte a bis f für Ziel-Genom-DNA-Moleküle durchgeführt werden, wobei:
- das in Schritt c) erhaltene Gemisch zusätzlich Ziel-Genom-DNA-Moleküle und Nicht-Ziel-Genom-DNA-Moleküle umfasst,
- in Schritt d) die Ziel-Genom-DNA-Moleküle an die Capture-Oligonukleotide für Ziel-Genom-DNA-Moleküle hybridisiert werden, wodurch Ziel-Genom-DNA-Moleküle erhalten werden, die an den festen Träger gebunden sind,
- in Schritt e) zusätzlich die Nicht-Ziel-Genom-DNA-Moleküle von den Ziel-Genom-DNA-Molekülen, die an den festen Träger gebunden sind, getrennt werden,
- in Schritt f) doppelsträngige Nukleinsäuren aus den Ziel-Genom-DNA-Molekülen durch Nukleinsäuresynthese erzeugt werden, wobei die Capture-Oligonukleotide für Genom-DNA-Moleküle als Primer und die Ziel-Genom-DNA-Moleküle als Templates dienen.

4. Verfahren nach Anspruch 3, **dadurch gekennzeichnet, dass** die Ziel-Genom-DNA-Moleküle vor der Hybridisierung (Schritt d) fragmentiert und in Einzelstränge dissoziiert wurden.

5. Verfahren nach Anspruch 4, **dadurch gekennzeichnet, dass** die fragmentierten genomischen DNA-Moleküle eine Länge von 50-10.000 Nukleotiden aufweisen.

6. Verfahren nach Anspruch 3 bis 5, **dadurch gekennzeichnet, dass** Schritt g) zusätzlich für die Ziel-Genom-DNA-Moleküle durchgeführt wird, mittels
g) Anfügen der Barcode-Oligonukleotide an die doppelsträngigen Nukleinsäuren aus den Ziel-Genom-DNA-Molekülen, wodurch barcodierte Nukleinsäuren aus den Ziel-Genom-DNA-Molekülen erzeugt werden.

7. Verfahren nach Anspruch 6, **dadurch gekennzeichnet, dass** für Ziel-Genom-DNA-Moleküle die Barcode-Oligonukleotide in Schritt (g) durch Ligation angefügt werden.

8. Verfahren nach einem der Ansprüche 1-7, **dadurch gekennzeichnet, dass** die Capture-Oligonukleotide für Ziel-RNA-Moleküle, die Capture-Oligonukleotide für Ziel-DNA-Moleküle und die Barcode-Oligonukleotide über einen Linker an den festen Träger gebunden sind, wobei der Linker nach Schritt g) gespalten wird, wodurch die Nukleinsäuren vom festen Träger abgespalten werden.

9. Verfahren nach Anspruch 10, **dadurch gekennzeichnet, dass** der Linker enzymatisch gespalten wird.

10. Verfahren nach einem der Ansprüche 8 bis 9, **dadurch gekennzeichnet, dass** die Capture-Oligonukleotide für Ziel-RNA-Moleküle, die Capture-Oligonukleotide für Ziel-DNA-Moleküle und die Barcode-Oligonukleotide nach Schritt g) durch eine ortsspezifische Endonuklease gespalten werden, wodurch die Nukleinsäuren von dem festen Träger abgespalten werden.

11. Verfahren nach einem der Ansprüche 1 bis 2, **dadurch gekennzeichnet, dass** die Capture-Oligonukleotide für Ziel-Genom-DNA-Moleküle als Oligonukleotide zur Gesamtgenom-Amplifikation dienen und über einen abspaltbaren Linker an den festen Träger gebunden sind und
dass nach Schritt c) eine Gesamtgenom-Amplifikation der Ziel-Genom-DNA-Moleküle durchgeführt wird, die die folgenden Schritte umfasst:
a. Abspalten der Oligonukleotide zur Gesamtgenom-Amplifikation von dem festen Träger,
b. Durchführen einer Gesamtgenom-Amplifikation unter Verwendung einer Polymerase mit Strangverdrängungsaktivität, wobei die Oligonukleotide zur Gesamtgenom-Amplifikation als Primer dienen, wodurch amplifizierte Ziel-Genom-DNA-Fragmente erhalten werden.

12. Verfahren nach einem der Ansprüche 1 bis 11, **dadurch gekennzeichnet, dass** die Zielnukleinsäuren aus einer Gruppe ausgewählt sind, bestehend aus Nukleinsäuren, die für T-Zell-Rezeptorketten oder B-Zell-Rezeptorketten kodieren.

13. Verfahren nach einem der Ansprüche 1 bis 12, **dadurch gekennzeichnet, dass** mindestens 50 % der Partitionen einen festen Träger und eine Zelle umfassen.

## Revendications

1. Procédé de génération d'acides nucléiques cibles à code-barres à partir d'une pluralité de cellules comprenant les étapes :
a. fournir une pluralité de cellules comprenant des molécules d'ARN cibles et au moins un support solide comprenant des oligonucléotides de capture pour lesdites molécules d'ARN cibles et des oligonucléotides code-barres
b. compartimenter ladite pluralité de cellules et lesdits supports solides de telle sorte que chaque cellule est incluse dans un compartiment distinct et que chaque compartiment comprend un support solide
c. lyser lesdites cellules, obtenant ainsi un mélange de molécules d'ARN cibles et non cibles
d. hybrider lesdites molécules d'ARN cibles aux oligonucléotides de capture pour lesdites molécules d'ARN cibles, obtenant ainsi des molécules d'ARN cibles fixées audit support solide
e. rompre les compartiments et séparer les molécules d'ARN non cibles des molécules d'ARN cibles fixées audit support solide
f. générer des acides nucléiques double brin à partir des molécules d'ARN cibles par une synthèse d'acides nucléiques qui est une réaction de transcription inverse avec changement de matrice, les oligonucléotides de capture servant d'amorces, les molécules d'ARN cibles servant de matrices et les oligonucléotides code-barres servant d'oligonucléotides de changement de matrice
g. fixer les oligonucléotides code-barres aux acides nucléiques double brin issus de molécules d'ARN cibles, générant ainsi des acides nucléiques à code-barres issus de molécules d'ARN cibles
**caractérisé en ce que**, à l'étape a), ladite pluralité de cellules comprend en outre des molécules d'ADN génomique cibles et ledit au moins un support solide comprend en outre des oligonucléotides de capture pour les molécules d'ADN génomique cibles et **en ce que** les oligonucléotides de capture pour les molécules d'ARN cibles et d'ADN génomique cibles sont différents.

2. Procédé selon la revendication 1, **caractérisé en ce que** les oligonucléotides de capture pour les molécules d'ADN génomique cibles comprennent une séquence code-barres.

3. Procédé selon l'une quelconque des revendications 1 à 2, **caractérisé en ce que** les étapes a à f sont en outre réalisées pour les molécules d'ADN génomique cibles, dans lequel :
- le mélange obtenu à l'étape c) comprend en outre des molécules d'ADN génomique cibles et des molécules d'ADN génomique non cibles
- à l'étape d), lesdites molécules d'ADN génomique cibles sont hybridées aux oligonucléotides de capture pour les molécules d'ADN génomique cibles, obtenant ainsi des molécules d'ADN génomique cibles fixées audit support solide
- à l'étape e), les molécules d'ADN génomique non cibles sont en outre séparées des molécules d'ADN génomique cibles fixées audit support solide
- à l'étape f), des acides nucléiques double brin issus de molécules d'ADN génomique cibles sont générés par synthèse d'acides nucléiques, les oligonucléotides de capture pour les molécules d'ADN génomique servant d'amorces et les molécules d'ADN génomique cibles servant de matrices.

4. Procédé selon la revendication 3, **caractérisé en ce que** les molécules d'ADN génomique cibles sont fragmentées et dissociés en brins simples avant l'hybridation (étape d).

5. Procédé selon la revendication 4, **caractérisé en ce que** les molécules d'ADN génomique fragmentées ont une longueur de 50 à 10 000 nucléotides.

6. Procédé selon les revendications 3 à 5, **caractérisé en ce que** l'étape g) est en outre réalisée pour les molécules d'ADN génomique cibles par
g) fixer les oligonucléotides code-barres aux acides nucléiques double brin issus de molécules d'ADN génomique cibles, générant ainsi des acides nucléiques à code-barres issus de molécules d'ADN génomique cibles.

7. Procédé selon la revendication 6, **caractérisé en ce que** pour les molécules d'ADN génomique cibles, les oligonucléotides code-barres sont fixés à l'étape (g) par ligature.

8. Procédé selon l'une quelconque des revendications 1 à 7, **caractérisé en ce que** les oligonucléotides de capture des molécules d'ARN cibles, les oligonucléotides de capture des molécules d'ADN cibles et les oligonucléotides code-barres sont liés audit support solide par un lieur, ledit lieur étant clivé après l'étape g), libérant ainsi lesdits acides nucléiques dudit support solide.

9. Procédé selon la revendication 10, **caractérisé en ce que** le lieur est clivé enzymatiquement.

10. Procédé selon l'une quelconque des revendications 8 à 9, **caractérisé en ce que** les oligonucléotides de capture pour les molécules d'ARN cibles, les oligonucléotides de capture pour les molécules d'ADN cibles et les oligonucléotides code-barres sont clivés après l'étape g) par une endonucléase spécifique d'un site, libérant ainsi lesdits acides nucléiques dudit support solide.

11. Procédé selon l'une quelconque des revendications 1 à 2, **caractérisé en ce que** les oligonucléotides de capture pour les molécules d'ADN génomique cibles servent d'oligonucléotides d'amplification du génome entier et sont fixés au support solide par un lieur détachable et **en ce qu'**après l'étape c), une amplification du génome entier de molécules d'ADN génomique cibles est réalisée, comprenant les étapes :
a. libérer les oligonucléotides d'amplification du génome entier dudit support solide
b. réaliser une amplification du génome entier en utilisant une polymérase avec activité de déplacement de brin, lesdits oligonucléotides d'amplification du génome entier servant d'amorces, obtenant ainsi des fragments d'ADN génomique cibles amplifiés.

12. Procédé selon l'une quelconque des revendications 1 à 11, **caractérisé en ce que** les acides nucléiques cibles sont choisis dans un groupe constitué par les acides nucléiques codant pour : les chaînes du récepteur des lymphocytes T ou les chaînes du récepteur des lymphocytes B.

13. Procédé selon l'une quelconque des revendications 1 à 12, **caractérisé en ce qu'**au moins 50 % des compartiments comprennent un support solide et une cellule.
